Europäisches Patentamt

European Patent Office

Office européen des brevets

(19)

(11) Numéro de publication: **0 230 829**
**A1**

## DEMANDE DE BREVET EUROPEEN

(12)

(21) Numéro de dépôt: **86402867.5**

(22) Date de dépôt: **19.12.86**

(51) Int. Cl.4: **D21H 3/02** , G09F 5/04 ,
A61K 7/46

(30) Priorité: **31.12.85 FR 8519490**

(43) Date de publication de la demande:
**05.08.87 Bulletin 87/32**

(84) Etats contractants désignés:
**AT BE CH DE ES GB GR IT LI LU NL SE**

(71) Demandeur: **YLANG**
**92 route de Cannes**
**F-06130 Grasse(FR)**

(72) Inventeur: **Zelter, Jean-Claude Etienne**
**8 avenue Etienne Carémil**
**Grasse (Alpes Maritimes)(FR)**

(74) Mandataire: **Herrburger, Pierre**
**Cabinet Pierre Herrburger 115, boulevard**
**Haussmann**
**F-75008 Paris(FR)**

(54) **Support de papier, notamment pour le stockage de parfums.**

(57) Support de papier (1) tel que carte parfumée, encart publicitaire, papier non-tissé ou autres.
caractérisé en ce que des microcapsules de stockage (2) du parfum sont noyées dans la masse au moment de la préparation de la pâte.

Le support de papier (1) comporte en outre des microcapsules neutres (3) remplies d'air ou de liquide, de diamètre plus important qui font office d'éléments amortisseurs pour proléger les microcapsules de stockage (2) du parfum contre l'écrasement.

EP 0 230 829 A1

## " Support de papier notamment pour le stockage de parfums"

L'invention concerne un support de papier tel que carte parfumée, encart publicitaire, papier non-tissé ou autres, destiné notamment à recevoir une petite quantité de parfum stockée dans des microcapsules susceptibles d'être écarées par frottement, pour libérer l'arôme du parfum.

On connaît déjà des supports de papier tels que des encarts publicitaires qui sont parfumés par projection ou trempage dans des parfums choisis. Ce type de carton parfumé, placé dans une revue ou un journal, dégage une forte odeur au début, puis cette odeur s'estompe et disparaît complètement, de telle sorte que le lecteur ne peut se rendre compte des qualités olfactives du parfum présenté.

On connaît également des encarts publicitaires formés d'une pochette dans laquelle est glissée une carte parfumée. L'odeur ainsi stockée par projection ou trempage dans le parfum, est un peu mieux protégée. Cependant, la fabrication d'un tel encart publicitaire est extrêmement longue et coûteuse.

On connaît également des cartons publicitaires ou des encarts suivant lesquels le parfum est stocké dans des microcapsules d'environ un dixième de millimètre, collées sous forme d'une couche sur le carton-support. La couche de microcapsules contenant le parfum est protégée par un rabat de protection. Cette solution présente l'avantage de conserver intactes les qualités olfactives du parfum lorsque l'encart publicitaire arrive chez le lecteur. Ce dernier soulève le rabat et par frottement ou grattage, écrase les microcapsules qui libèrent ainsi le parfum stocké. Ce type d'encart publicitaire est cependant extrêmement difficile à manipuler et demande une fabrication extrêmement longue, comprenant de nombreuses opérations. C'est ainsi que le support de carton doit être enduit d'une couche de colle, puis on met en place la couche de microcapsules par soufflage de ces capsules sur le couche de colle. On recouvre, enfin, la couche de capsules d'un rabat de protection. Malgré ce rabat de protection, les capsules sont fragilisées du fait qu'elles sont superposées sur le support en carton.

En outre, la dimension de ces microcapsules, d'environ un dixième millimètre, détermine également une plus grande fragilité.

La présente invention a donc pour but de créer un support de papier tel que carte parfumée, encart publicitaire, papier non tissé ou autres, ne présentant aucune odeur avant écrasement des capsules, préservant ainsi les qualités olfactives du parfum stocké, lorsque l'encart publicitaire parvient à son destinataire.

La présente invention a pour but également de créer un support de papier permettant la présentation de plusieurs essences parfumées dans un même journal.

La présente invention a également pour but de créer un support de papier permettant de préservser les microcapsules de stockage du parfum contre tout phénomène d'écrasement du cours de la fabrication des journaux ou de leur distribution.

Enfin, la présente invention a pour but de créer un support de papier présentant des essences parfumées à un prix de revient extrêmement faible, tout en permettant de tester, dans les meilleures conditions, les qualités olfactives du parfum stocké et présenté.

A cet effect, l'invention concerne un support de papier tel que carte parfumée, encart publicitaire, papier non tissé ou autres, destiné notamment à recevoir une petite quantité de parfum stockée dans des microcapsules susceptibles d'être écrasées par frottement pour libérer l'arôme du parfum, support de papier caractérisé en ce que les microcapsules de stockage du parfum sont noyées dans la masse au moment de la préparation de la pâte.

Suivant une autre caractéristique de l'invention, des microcapsules neutres d'un diamètre supérieur à celui des microcapsules de stockage du parfum, sont également noyées dans la masse du support de papier au moment de la préparation de la pâte et font office d'éléments amortisseurs pour protéger les microcapsules de stockage du parfum contre l'écrasement.

Suivant une autre caractéristique de l'invention, les microcapsules de stockage du parfum sont des petites billes creuses de gélatine présentant un diamètre de l'ordre de 5 microns.

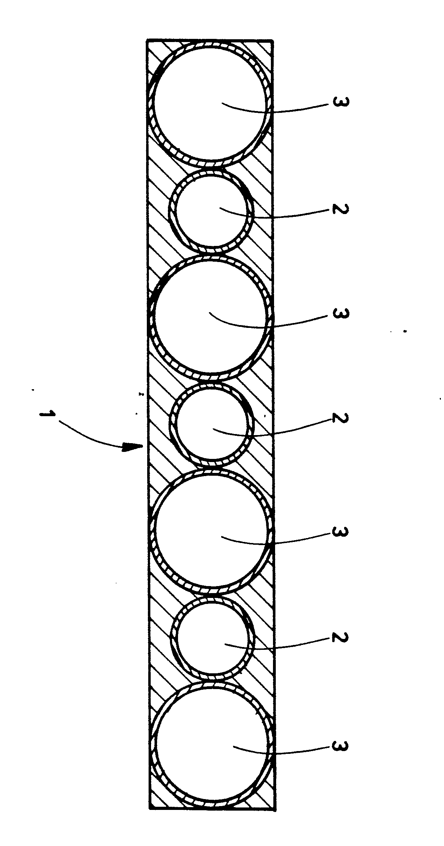

La présente invention sera mieux comprise à l'aide d'un mode de réalisation du spport de papier, représenté schématiquement, à titre d'exemple non limitatif sur l'unique figure jointe qui est :
-une vue en coupe du support de papier contenant les microcapsules de stockage de parfum et les microcapsules neutres faisant office d'éléments amortisseurs.

Selon la figure, le support de papier 1 représente, en coupe, une carte parfumée, un encart publicitaire ou tout autre support de présentation, notamment de parfum. Il est possible d'imaginer la réalisation d'un papier non-tissé tel que des mouchoirs jetables. Le support de papier 1 comporte des microcapsules de stockage de parfum 2. Ces microcapsules de stockage de parfum sont noyées dans la masse au moment où l'on prépare la pâte à papier. Les microcapsules de stockage de parfum 2 sont de petites billes creuses de gélatine présentant un diamètre de l'ordre de 5 microns.

Le support de papier 1 comporte, en outre, des microcapsules neutres 3 remplies d'air ou de liquide, ne présentant aucune propriété olfactive. Ces microcapsules neutres 3 sont également des billes creuses de gélatine noyées dans la masse au moment de la préparation de la pâte à papier. Le diamètre des microcapsules 3 est très largement supérieur au diamètre des microcapsules de stockage de parfum 2.

Les microcapsules 3 de diamètre plus important font office d'éléments amortisseurs pour protéger les microcapsules de stockage de parfum contre tous les phénomènes d'écrasement dus aux différentes manipulations du support de papier 1.

L'originalité de l'invention est donc de noyer dans la masse des microcapsules contenant les essences de parfum ainsi que des microcapsules de diamètre plus important faisant office d'éléments amortisseurs contre les phénomènes d'écrasement.

Les microcapsules 2 permettent de conserver toutes les propriétés olfactives du parfum jusqu'à ce que l'utilisateur, par frottement ou grattage, détruise les microcapsules.

Les microcapsules de stockage de parfum 2 présentent un diamètre de l'ordre de 5 microns correspondant parfaitement à une inclusion dans la masse d'un support de papier d'un poids de 15 g au mètre carré.

Les microcapsules neutres 3 ont pour but de protéger les microcapsules 2 contre tout phénomènes d'écrasement, notamment lors de la fabrication du support de papier 1 qui, à une certaine étape du procédé de fabrication défile entre deux cylindres qui lui font subir une pression intense.

Il en est de même lorsque le support de papier 1 est soumis à l'action des presses à imprimer.

Les termes "support de papier" sont à prendre au sens le plus large, c'est-à-dire "toute matière fabriquée par une technique papetière".

## Revendications

1°) Support de papier tel que carte parfumée, encart publicitaire, papier non-tissé, ou autres, destiné notamment à recevoir une petite quantité de parfum stockée dans les microcapsules susceptibles d'être écrasées par frottement pour libérer l'arôme du parfum, support de papier caractérisé en ce que les microcapsules de stockage (2) du parfum sont noyées dans la masse au moment de la préparation de la pâte.

2°) Support de papier conforme à la revendication 1, caractérisé en ce que les microcapsules neutres (3) d'un diamètre supérieur à celui des microcapsules de stockage (2) du parfum sont également noyées dans la masse du support de papier (1) au moment de la préparation de la pâte et font office d'éléments amortisseurs pour protéger les microcapsules de stockage (2) du parfum contre l'écrasement.

3°) Support de papier conforme aux revendications 1 et 2 précédentes, caractérisé en ce que les microcapsules de stockage (2) du parfum sont des petites billes creuses de gélatine présentant un diamètre de l'ordre de 5 microns.

0 230 829

3   2   3   2   3   2   3

1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 066 821 (C. FREUDENBERG) <br> * En entier et en particulier page 1, paragraphe 2; page 2, lignes 16-20 * <br><br> --- | 1 | D 21 H   3/02 <br> G 09 F   5/04 <br> A 61 K   7/46 |
| A | FR-A-2 012 563 (MINNESOTA MINING AND MANUFACTURING CO.) <br> * En entier * <br><br> ----- | 1 | |

DOMAINES TECHNIQUES
RECHERCHES (Int. Cl.4)

A 61 K
D 21 H
G 09 F

Le présent rapport de recherche a été établi pour toutes les revendications

| Lieu de la recherche | Date d'achèvement de la recherche | Examinateur |
|---|---|---|
| LA HAYE | 24-03-1987 | NESTBY K. |